# EUROPEAN PATENT APPLICATION

(11) **EP 0 553 424 A1**
(43) Date of publication of application: **04.08.1993**
(21) Application number: 92120052.3
(22) Date of filing: 25.11.1992
(51) Int. Cl.: A61B 17/58

(54) **Spinal column retaining apparatus**

(30) Priority: 30.01.1992 US 828030
(71) Applicant: ACROMED CORPORATION, Cleveland Ohio 44115 (US)
(72) Inventor: Asher, Marc A., Kansas 66211 (US); Strippgen, Walter E., Colorado 80403 (US); Heinig, Charles F., Virginia 23178 (US); Carson, William L., Missouri 65203 (US); Stahurski, Terrence, Ohio 44116 (US)
(74) Representative: Rottmann, Maximilian R.

(57) **Abstract**

An apparatus is provided to retain vertebrae of a spinal column in a desired spatial relationship. A plurality of fasteners (20) are connected with the plurality of vertebrae. A longitudinal member (26) is releasably connected with the fasteners by a plurality of connector means (30). The longitudinal member and fasteners are movable relative to each other to obtain the desired spatial relationship between the longitudinal member and the vertebrae to which the fasteners are connected. The connector means enables the longitudinal member, located in opening (36), to be positioned about an axis spaced from the fastener and at an angle, preferably perpendicular to the axis of the longitudinal member. The connector means also enables the distance between the longitudinal member and the fastener to be varied. Once the longitudinal member and fasteners have been positioned in the desired spatial relationship, clamps (32) are engaged to hold the longitudinal member, fasteners and connector means against relative movement.

## Description

### Background of the Invention

The present invention relates to an apparatus to retain vertebrae of a spinal column in a desired spatial relationship.

A known apparatus for retaining vertebrae in a desired spatial relationship is disclosed in U.S. Patent No. 4,648,388. The apparatus disclosed in the '388 patent includes a plurality of threaded fasteners (bone screws) which are connected with vertebrae of a spinal column. Retaining rods are bent to a desired configuration as a function of the desired spatial relationship between the vertebrae of the spinal column. After a rod has been bent to the desired configuration, it is inserted into clamps connected with the fasteners. The clamps are then clamped to the rod to hold the vertebrae against movement relative to the rod. Other known apparatus for retaining vertebrae in a desired spatial relationship are disclosed in U.S. Patent Nos. 4,611,581; 4,655,199; 4,887,595; and 5,024,213. With this known apparatus, once the threaded fasteners have been connected with the vertebrae, it is sometimes difficult to adjust the position of a retaining rod relative to the fasteners to match certain desired anatomic positions.

### Summary of the Invention

The present invention provides a new and improved apparatus for use in retaining vertebrae of a spinal column in a desired spatial relationship. The apparatus includes a fastener having a threaded end portion which engages a vertebra of the spinal column. A connector means interconnects the fastener and a longitudinal member, such as a rod. The connector means is adjustable to enable the relative positions of the longitudinal member and the fastener to be varied while the fastener remains stationary relative to the vertebra.

The connector means enables the longitudinal member to be positioned about an axis spaced from the fastener and extending at an angle preferably perpendicular to the axis of the longitudinal member. The connector means further enables the distance between the longitudinal member and the fastener to be varied. Therefore, the relative positions of the fastener and the longitudinal member can be varied in diverse ways to obtain the desired spatial relationship between the fastener and the longitudinal member.

The connector means includes a clamp means and a connector rod member. The clamp means is secured to a vertebrae by the fastener. The clamp means clamps the connector rod member of the connector means to hold the connector rod member in a desired position. The connector rod member receives and fixes the longitudinal member in place. The connector rod member can pivot about its axis which extends at an angle, preferably perpendicular, to the axis of the longitudinal member when the clamp means is in a released condition. The connector rod member can also move laterally relative to the clamp means to adjust the distance between the fastener and the longitudinal member when the clamp means is in a released condition.

### Brief Description of the Drawings

Further features of the present invention will become apparent to those skilled in the art to which the present invention relates from reading the following specification with reference to the accompanying drawings, in which:
Fig. 1 is a view of a corrective device connected with a portion of a spinal column by retainer assemblies constructed in accordance with the present invention to maintain a desired spatial relationship between vertebrae of the spinal column;
Fig. 2 is a view taken along the line 2-2 in Fig. 1;
Fig. 3 is a sectional view illustrating the manner in which a retainer assembly is connected with a vertebra;
Fig. 4 is a pictorial illustration of the retainer assembly used in connecting the spinal corrective device to the spinal column in Fig. 1;
Fig. 5 is an enlarged pictorial illustration of a clamp means which forms a part of the retainer assembly of Fig. 4;
Fig. 6 is an enlarged pictorial illustration of a connector member which forms a part of the retainer assembly of Fig 4; and
Fig. 7 is a pictorial illustration of a modified embodiment of the present invention.

### Description of a Preferred Embodiment of the Present Invention

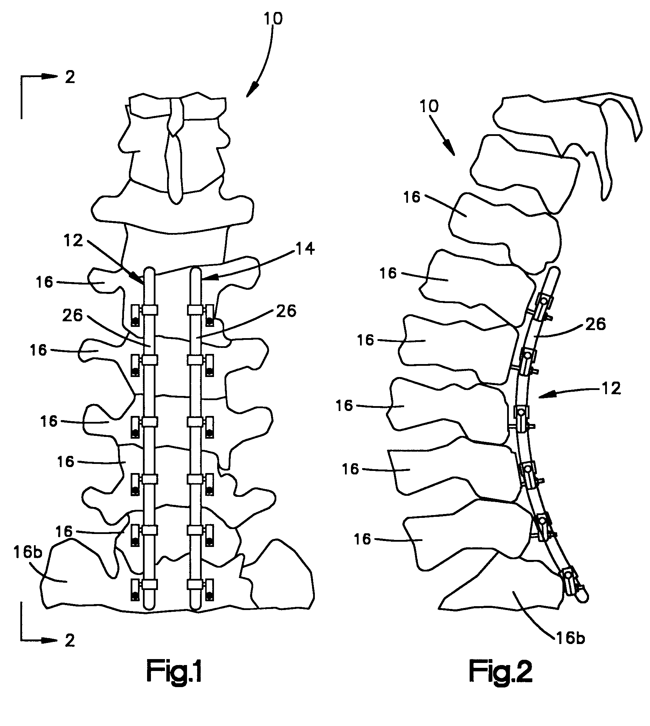

A spinal column 10 to which a pair of retainer assemblies 12 and 14 are connected is illustrated in Figs. 1 and 2. The retainer assemblies 12 and 14 retain vertebrae 16 and sacrum 16b of the spinal column in a desired spatial relationship relative to each other.

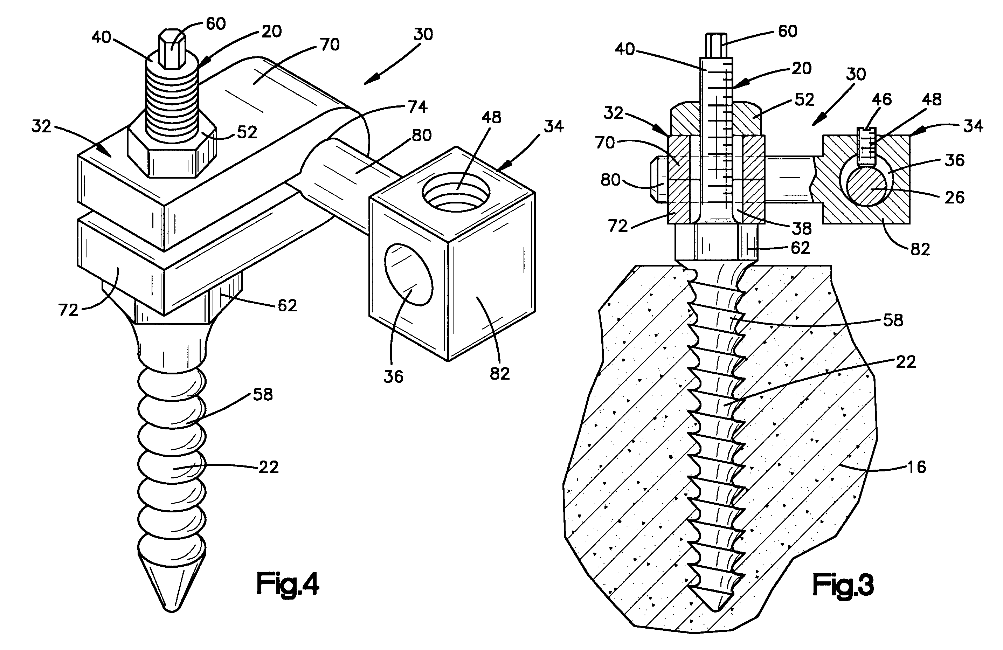

The retainer assemblies 12 and 14 have the same construction and include fasteners 20 (Fig. 3) made of a bio-compatible material, such as stainless steel. The fasteners 20 have threaded inner end portions 22 which engage the vertebrae 16 and sacrum 16b and fixedly secure the fasteners in the vertebrae and sacrum. One fastener 20 is shown in Fig. 3.

Each of the retainer assemblies 12 and 14 includes a longitudinal member such as a cylindrical rod 26 which extends along the spinal column. The rod 26 is made of a bio-compatible material such as stainless steel. Each of the rods 26 has a length which is at least sufficient to enable the rod to span at least two of the vertebrae 16 or sacrum 16b and vertebrae 16. In the embodiment of the invention illustrated in Figs. 1 and 2, the rods 26 span five vertebrae 16 and sacrum 16b. Of course, the length of the rods in any particular installation will depend upon the condition to be corrected and the number of vertebrae 16 to be held in a desired spatial relationship relative to each other by the retainer assemblies 12 and 14. The rods 26 are bent to conform to a desired curvature of the spinal column 10 in all or any of the three possible anatomic planes.

A plurality of connector means 30 interconnect the rods 26 and the fasteners 20 (Fig. 3). Each of the connector means 30 includes a clamp means 32 (Figs. 3-5) and a connector rod member 34 (Figs. 3, 4 and 6). The connector rod member 34 is provided with a first opening 36 through which the rod 26 extends. A set screw 46 (Fig. 3) engages an internally threaded opening 48 in the connector rod member 34. The set screw 46 clamps the rod 26 against a side surface of the opening 36 when a desired spatial relationship between the fastener 20 and the rod has been achieved. The surface defining the opening 36 may have a pair of spaced projections which engage and clamp against spaced portions of the rod 26, as shown in U.S. Patent 5,024,213.

Each of the clamp means 32 (Fig. 3) has an opening 38 through which an outer end portion 40 of the fastener 20 extends. A clamp nut 52 engages threads on the outer end portion 40 of the fastener 20. The clamp nut 52 clamps the clamp means 32 against an intermediate portion 62 of the fastener 20 that has wrenching flats. The clamp nut 52 clamps the connector means 30 in position once the fastener 20 and rod 26 are positioned relative to each other.

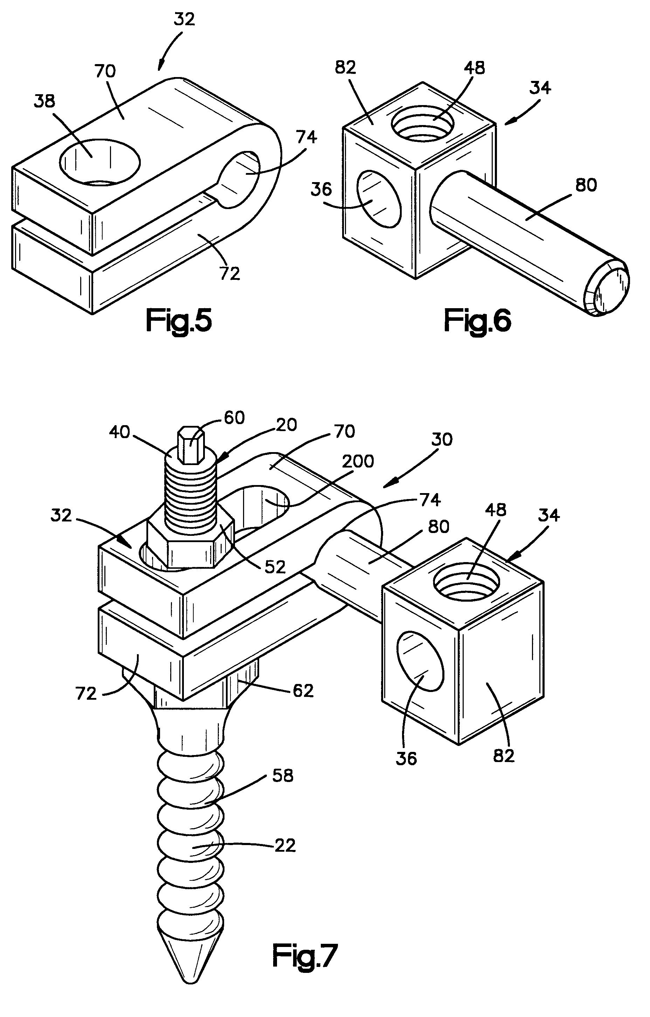

The clamp means 32 of connector means 30 (Figs. 4 and 5) includes a first clamp half 70 and a second clamp half 72. The first and second clamp halves 70 and 72 define an opening 74 through which the connector rod member 34 extends. The connector rod member 34 can pivot in the opening 74 to position the rod 26 about the axis of the opening 74 when the clamp means 32 is released. The connector rod member 34 can also slide in the opening 74 to adjust the distance between the rod 26 and the fastener 20, when the clamp means 32 is in a released condition. When clamp nut 52 is tightened onto the fastener 20, the clamp means 32 clamps the connector rod member 34 in position.

The drawings illustrate the clamp means 32 as one piece. However, the clamp means 32 could be two separate pieces which when tightened together clamp against the connector rod member 34.

The connector rod member 34 (Fig. 6) includes a rod portion 80 which extends from a block portion 82. The opening 36 extends through the block portion 82. The axis of the rod portion 80 extends at an angle, preferably perpendicular, to the axis of the opening 36.

The rod portion 80 of the connector rod member 34 engages the clamp means 32 in the opening 74 (Fig. 4). The connector rod member 34 can pivot about the axis of the rod portion 80 to position the rod 26 about the axis of the opening 74. Also, the rod portion 80 can slide in the opening 74 to adjust the distance between the rod 26 and the fastener 20.

When the retainer assemblies 12 and 14 are to be mounted on a spinal column 10 (Figs. 1 and 2), a plurality of fasteners 20 (Fig. 3) are connected to the vertebrae 16. This is accomplished by turning or screwing coarse thread convolutions 58 on the inner end portions 22 of the fasteners 20 into the vertebrae. Torque is applied to the fasteners 20 at wrenching flats 60 on the outer ends of the fasteners or at the wrenching flats at the intermediate portions 62 of the fasteners. Once the helical thread convolution 58 on the inner end portion of the fastener 20 has been turned into the bony material of a vertebra 16, in the manner shown in Fig. 3, the fastener 20 is fixedly connected with a vertebra 16 and does not move relative to the vertebra.

After the rods 26 have been bent to the desired configuration in the necessary anatomic planes, a plurality of connector means 30 are placed on each of the rods by inserting the rods through the openings 36 of the connector rod members 34. Once a number of connector means 30 corresponding to the number of vertebrae and or sacrum locations to which the rods 26 are to be connected have been positioned on each of the rods, the rods are positioned along the spinal column 10 with a longitudinal central axis of the rods extending generally parallel to the longitudinal central axis of the spinal column. Thus, the rods 26 are generally located in the position shown in Fig. 1 relative to the spinal column 10.

Once the rods 26 have been positioned relative to the spinal column 10, the openings 38 in the clamp means 32 are positioned in engagement with the outer end portions 40 of the fasteners 20. As this is done, the clamp nuts 52 are loosely threaded onto the outer end portions 40 of the fasteners 20 to prevent disengagement of the clamp means 32 from the outer end portions of the fasteners.

The spatial relationship between each of the rods 26 and the associated fasteners 20 is then adjusted. The rod 26 is positioned about the axes of the rod portion 80 of the connector rod member 34 and the opening 74 in the clamp means 32. The distance between the rod 26 and the fastener 20 is adjusted by sliding the rod portion 80 in the opening 74.

Once the fastener 20 and the rod 26 have been located at a desired spatial relationship relative to each other, the clamp nut 52 is tightened to hold the rod 26 and the fastener 20 in the desired spatial relationship. Once the clamp nut 52 has been tightened to securely hold the connector means 30 against movement relative to the fastener 20, the set screw 46 is tightened to clamp the connector means 30 against movement relative to the rod 26. Tightening the set screw 46 firmly presses the rod 26 into the bottom of opening 36 (Fig. 3). This results in the connector means 30 being firmly clamped against movement relative to the rod 26.

The rod 80 and the surface defining the opening 74 in the clamp means 32 may have projections which positively interlock when the clamp means 32 clamps against the rod 80. These interlocking projections would positively prevent relative rotation of the rod 80 about its axis relative to the clamp means 32.

Figure 7 illustrates a further embodiment of the present invention. The Fig. 7 embodiment is substantially identical to the embodiment of Fig. 4 and similar reference numbers are used in Fig. 7 and Fig. 4. The embodiment of Fig. 7 includes an elongate slot 200 in the clamp member 32. The screw 20 is received in the slot 200, and the slot 200 permits relative adjustment of the screw 20 and clamp means 32 and connector rod member 34.

From the above description of the invention, those skilled in the art will perceive improvements, changes and modifications in the invention. Such improvements, changes and modifications within the skill of the art are intended to be covered by the appended claims.

## Claims

1. An apparatus for use in retaining vertebrae of a spinal column in a desired spatial relationship, said apparatus comprising a fastener for engaging the vertebra, a longitudinal member which is positionable along the spinal column, connector means for releasably interconnecting said longitudinal member and said fastener for positioning said fastener and said longitudinal member in the desired spatial relationship, said connector means enabling said longitudinal member to be positioned about an axis which is spaced from said fastener and extends at an angle to the axis of said longitudinal member when said connector means is in a released condition.

2. An apparatus as set forth in claim 1 wherein said angle is 90°.

3. An apparatus as set forth in claim 1 wherein said connector means enables the distance between said longitudinal member and said fastener to be varied.

4. An apparatus as set forth in claim 1 wherein said connector means includes clamp means which is engagable to maintain the desired position of said longitudinal member relative to said fastener.

5. An apparatus as set forth in claim 4 wherein said connector means further includes a connector rod member which extends from said clamp means and is connectable with said longitudinal member, said connector rod member being pivotable relative to said clamp means when said clamp means is in a released condition.

6. An apparatus as set forth in claim 5 wherein said clamp means comprises first and second clamp halves defining an opening, said connector rod member extending into the opening defined by said first and second clamp halves.

7. An apparatus as set forth in claim 6 wherein said first clamp half has surface means defining a first opening and said second clamp half has surface means defining a second opening, said fastener being extendable through the first and second openings in said first and second clamp halves, said first and second clamp halves being clamped between an intermediate portion on said fastener and a nut threadably engagable with said fastener.

8. An apparatus as set forth in claim 6 wherein said connector rod member includes a rod portion extendable into the opening defined by said first and second clamp halves and which is rotatable in the opening about said axis.

9. An apparatus for use in retaining vertebrae of a spinal column in a desired spatial relationship, said apparatus comprising a fastener having a threaded end portion for attachment to a vertebra, a longitudinal member which is positionable along the spinal column, and a connector means for interconnecting said longitudinal member and said fastener, said connector means comprising a clamp means and a connector rod member which is releasably clamped by said clamp means and extends from said clamp means to said longitudinal member, said connector rod member being pivotable relative to said clamp means and having means connectable with said longitudinal member.

10. An apparatus as set forth in claim 9 wherein said clamp means comprises first and second clamp halves, said connector member extending into an opening defined by said first and second clamp halves.

11. An apparatus asset forth in claim 9 wherein said connector rod member includes a rod portion that is engagable with said clamp means and a block portion connectable with said longitudinal member, said connector rod member being pivotable about the axis of said rod portion when said clamp means is released.

12. An apparatus as set forth in claim 11 wherein said block portion has a passage through which said longitudinal member extends, and means for fixing said longitudinal member in said opening.
